# FASCICULE DE BREVET EUROPEEN

(11) **EP 4 074 270 B1**
(45) Date de publication et mention de la délivrance du brevet: **07.01.2026**
(21) Numéro de dépôt: 22170012.3
(22) Date de dépôt: 31.03.2020
(51) Int. Cl.: A61B 17/34, A61B 34/30, A61B 90/11, A61M 5/32, A61B 34/20, A61B 90/00

(54) **DISPOSITIF DE GUIDAGE D'UNE AIGUILLE MÉDICALE**
FÜHRUNGSVORRICHTUNG FÜR EINE MEDIZINISCHE NADEL
DEVICE FOR GUIDING A MEDICAL NEEDLE

(30) Priorité: 04.04.2019 FR 1903636
(43) Date de publication de la demande: 19.10.2022
(62) Demande divisionnaire de: 20715872.6
(73) Titulaire: Quantum Surgical, 34000 Montpellier (FR)
(72) Inventeur: NAHUM, Bertin, 34170 Castelnau-le-Lez (FR); BADANO, Fernand, 69006 Lyon (FR); BLONDEL, Lucien, 34070 Montpellier (FR); OLIVE, Sébastien, 34000 MONTPELLIER (FR)
(74) Mandataire: Santarelli

(56) Documents cités:
- EP-A1- 3 054 862
- EP-A1- 3 318 213
- WO-A1-2013/192598

## Description

### Domaine technique de l'invention

L'invention relève des équipements médicaux et s'inscrit dans le domaine des matériels médicaux montés à l'extrémité d'un bras robotisé d'assistance médicale. La présente invention concerne plus particulièrement un dispositif de guidage d'une aiguille médicale destiné à être fixé à un porte-outil d'un bras robotisé.

### Technique antérieure

Les interventions chirurgicales effectuées par la technique chirurgicale connue sous la dénomination « chirurgie mini-invasive » permettent à un opérateur, typiquement un chirurgien, d'atteindre une zone anatomique cible d'un patient par l'insertion d'instruments longs et fins dans une incision de faible longueur, par exemple de l'ordre du centimètre, réalisée dans le corps du patient.

Dans le cadre de certaines opérations, ces instruments peuvent être une ou des aiguilles ou encore des instruments rigides cylindriques (par exemple, antenne, électrode, canule) destinés à être insérés dans le corps d'un patient jusqu'à une certaine profondeur pour atteindre la zone anatomique cible.

Lorsque l'insertion de l'aiguille est entièrement réalisée manuellement par l'opérateur, le résultat de l'intervention est fortement dépendant de son habileté. Il est difficile d'atteindre une précision élevée et le risque d'erreurs médicales résultant de ce manque de précision, pouvant occasionner des blessures au patient, est important.

La précision de l'opération peut être améliorée grâce à l'utilisation de bras robotisés télécommandés par un opérateur. Ce type de bras robotisés reste partiellement dépendant de l'habileté de l'opérateur et peut nécessiter d'imager le patient en continu, ce qui contraint le patient à être soumis à une certaine dose d'irradiations.

Afin d'améliorer encore la précision du geste d'insertion et de limiter les doses d'irradiations auxquelles sont soumis le patient et le personnel médical, il est possible d'utiliser un bras robotisé commandé automatiquement.

Le bras robotisé porte à son extrémité un dispositif de guidage d'une aiguille.

L'opérateur renseigne au bras robotisé une coordonnée d'une zone anatomique cible d'un patient à atteindre et le bras est commandé de sorte à déplacer le dispositif de guidage d'une aiguille en regard de la zone anatomique cible. L'aiguille est ensuite insérée par un opérateur dans le dispositif de guidage de sorte à atteindre la zone anatomique cible. Pour que cette zone soit atteinte avec précision par l'aiguille, il est nécessaire de contrôler la position de l'axe de translation de l'aiguille au sein du dispositif de guidage. Il est donc indispensable de maitriser tout déplacement du dispositif de guidage et donc du bras.

Le contrôle du déplacement est d'autant plus important lorsque l'intervention nécessite l'insertion séquentielle d'aiguilles dans la zone anatomique cible. Dans ce cas, le dispositif de guidage d'une aiguille doit, pendant l'insertion de chaque aiguille, être immobile de sorte que la position de l'axe de translation de ladite aiguille soit conservée, puis permettre son relâchement une fois qu'elle est insérée dans la zone anatomique cible sans la déplacer. En effet, tout déplacement incontrôlé de l'aiguille une fois qu'elle est insérée dans la zone anatomique cible est susceptible de blesser le patient.

Il existe donc un besoin d'une part de guider l'insertion d'au moins une aiguille suivant un axe de translation dont la position est conservée jusqu'à ce qu'elle atteigne une zone anatomique cible, et d'autre part de libérer le dispositif de guidage de ladite aiguille, lorsque celle-ci est insérée dans la zone anatomique cible, sans risque de déplacer ladite aiguille.

Par ailleurs, il existe également un besoin de connaître la longueur de l'aiguille insérée dans le corps d'un patient.

Enfin, il existe un besoin de guider l'insertion d'au moins deux aiguilles de diamètre différents avec un même dispositif de guidage, au cours d'une même intervention médicale ou lors d'interventions médicales distinctes ; chaque aiguille devant être guidée suivant un même axe de translation dont la position est conservée jusqu'à ce qu'elle atteigne une zone anatomique cible et pouvant également être dégagée du dispositif de guidage lorsqu'elle est insérée dans la zone anatomique cible sans risque d'être déplacée.

### Présentation de l'invention

La présente invention a pour objectif de répondre aux besoins précités et concerne, à cet effet, un dispositif de guidage d'une aiguille comportant un porte-outil destiné à être fixé à l'extrémité d'un bras robotisé d'assistance médicale. Le porte-outil supporte un guide aiguille. Le guide aiguille comporte un premier mors et un second mors dotés respectivement d'une gorge, lesdites gorges s'étendant l'une et l'autre selon des axes longitudinaux parallèles. Lesdits premier mors et second mors sont supportés par le porte-outil de sorte à autoriser une mobilité en rotation des premier mors et second mors l'un par rapport à l'autre entre une position dite « position de guidage », dans laquelle les gorges sont attenantes et définissent un conduit de guidage d'une aiguille, et une position dite « position de dégagement » dans laquelle les gorges sont éloignées l'une de l'autre et définissent une zone de dégagement latéral d'une aiguille. De plus, le dispositif de guidage comporte un système de navigation optique destiné à être connecté à une unité de contrôle du bras robotisé déterminant, sur la base d'informations transmises par ledit système de navigation optique, la position des premier mors et second mors) l'un par rapport à l'autre.

Lorsque le dispositif de guidage occupe la position de guidage, une aiguille peut être guidée en translation à travers le conduit de guidage jusqu'à atteindre une zone anatomique cible d'un patient.

Le conduit de guidage est configuré de sorte à n'autoriser qu'un unique degré de liberté en translation à une aiguille.

Lorsque les mors sont en position de dégagement, le dispositif de guidage en translatant selon un sens opposé à la zone de dégagement est libre de dégager latéralement une aiguille ayant atteint une zone anatomique cible. Ce dégagement latéral intervient par exemple à la fin d'une opération médicale si une seule aiguille est à introduire dans la zone anatomique cible ou en cours d'opération médicale si une aiguille supplémentaire est à introduire dans la zone anatomique cible.

Il y a lieu de noter que dans le présent texte on mentionne par abus de langage que les positions de guidage et de dégagement sont occupées par le guide aiguille et par les mors.

Grace aux caractéristiques de la présente invention, une aiguille peut être dégagée du guide aiguille sans contact, de sorte qu'elle demeure fixe en position dans la zone anatomique cible.

Tout déplacement non souhaité de l'aiguille pouvant causer des blessures à un patient est ainsi évité.

Grâce à ces caractéristiques, le dispositif de guidage permet l'insertion successive d'une pluralité d'aiguilles de même diamètre avec un même dispositif de guidage, pour atteindre une zone anatomique cible, au cours d'une même opération médicale.

Dans des modes particuliers de réalisation, l'invention répond en outre aux caractéristiques suivantes, mises en œuvre séparément ou en chacune de leurs combinaisons techniquement opérantes.

Dans des modes particuliers de réalisation de l'invention, le premier mors ou le second mors comprend une poignée de manipulation sous la sollicitation de laquelle ledit premier mors ou second mors est entraîné en déplacement par rapport à l'autre mors.

Dans des modes particuliers de réalisation de l'invention, le porte-outil comprend un logement s'étendant longitudinalement entre deux ouvertures d'extrémité et au sein duquel est engagé le guide aiguille. Le porte-outil comporte également une ouverture axiale traversante s'étendant de l'une à l'autre desdites ouvertures d'extrémité selon un axe parallèle à l'axe longitudinal du logement, l'ouverture axiale traversante étant en vis-à-vis de la zone de dégagement latéral lorsque les mors sont en position de dégagement.

Ainsi, une fois qu'une aiguille a été guidée jusqu'à atteindre une zone anatomique cible, elle peut être dégagée du dispositif de guidage sans contact, de sorte qu'elle demeure fixe en position dans la zone anatomique cible.

Dans des modes particuliers de réalisation de l'invention, les premier mors et second mors sont formés par un mors fixe et par un mors mobile.

Dans des modes particuliers de réalisation de l'invention, le mors fixe est engagé dans le logement, ledit mors fixe et ledit logement présentant, sur leurs surfaces respectives en vis-à-vis, des éléments d'emboitement coopérant l'un avec l'autre pour immobiliser ledit mors fixe en rotation par rapport au porte-outil.

Les éléments d'emboitement peuvent coopérer directement l'un avec l'autre ; c'est le cas par exemple si les éléments d'emboitement sont respectivement formés par une rainure et une languette. Les éléments peuvent alternativement coopérer indirectement l'un avec l'autre ; c'est le cas par exemple si les éléments d'emboitement sont formés par des rainures dans lesquelles sont insérées une clavette ou une goupille.

Les éléments d'emboitement jouent avantageusement un rôle de détrompeur lors de l'insertion du guide aiguille dans le logement.

Dans des modes particuliers de réalisation de l'invention, les éléments d'emboitement sont respectivement formés par des reliefs de formes complémentaires dont :
- un relief s'étendant longitudinalement sur une surface dite « la surface externe » du mors fixe, et
- un relief s'étendant longitudinalement sur une surface dite « paroi interne » du logement.

Ces reliefs peuvent être formés par une languette et par une rainure.

Grâce aux éléments d'emboitement, l'immobilisation du mors fixe est assurée de façon simple et fiable.

Dans des modes particuliers de réalisation de l'invention, les premier mors et second mors sont fixés de manière amovible l'un à l'autre. Ils sont préférentiellement engagés dans le logement du porte-outil de manière amovible.

Une telle disposition vise notamment à augmenter l'efficacité de la stérilisation du dispositif de guidage, chaque pièce dudit dispositif pouvant être stérilisée individuellement.

Dans des modes particuliers de réalisation de l'invention, le mors fixe est formé d'un seul tenant avec le porte-outil ; le mors fixe et le porte-outil formant ainsi une pièce monobloc.

Dans des modes particuliers de réalisation de l'invention, le porte-outil comprend une lumière traversante s'étendant radialement au logement. Le premier ou le second mors comprend une poignée de manipulation s'étendant à travers la lumière et sous la sollicitation de laquelle ledit premier ou second mors est entraîné en déplacement dans le logement. La lumière forme avantageusement un chemin de guidage de la poignée de manipulation.

Dans des modes particuliers de réalisation de l'invention, la lumière comporte une portion s'étendant axialement par rapport au logement, ladite portion débouchant sur une surface du porte-outil dite « face supérieure » affleurante à l'une des ouvertures d'extrémité dudit logement.

Par conséquent, l'un des mors (éventuellement les deux mors, selon le mode particulier de réalisation de l'invention) peut être désolidarisé du porte-outil afin de stériliser chaque pièce du dispositif de guidage individuellement et ainsi, augmenter l'efficacité de la stérilisation du dispositif de guidage.

Grace à cette caractéristique, les pièces du dispositif de guidage peuvent également être remplacées individuellement.

Dans des modes particuliers de réalisation de l'invention, les premier mors et second mors présentent respectivement un épaulement axial. Les épaulements axiaux présentent des profils complémentaires par lesquels ils coopèrent l'un avec l'autre.

Les premier mors et second mors reposent l'un contre l'autre par leurs épaulements axiaux, lesdits épaulements étant imbriqués au moins partiellement l'un avec l'autre.

Cette caractéristique permet avantageusement de favoriser le guidage en rotation du second mors au sein du logement.

Avantageusement, les épaulements axiaux peuvent constituer une butée de débattement angulaire du second mors.

Dans des modes particuliers de réalisation de l'invention, les premier mors et second mors sont liés entre eux de manière amovible par l'intermédiaire d'une tige s'étendant longitudinalement depuis l'un des premier mors et second mors à travers un logement axial formé dans l'épaulement axial de l'autre mors. Les mors sont liés entre eux par une liaison mécanique n'autorisant qu'un degré de liberté en rotation.

Grâce à la tige, les axes de rotation des mors sont coaxiaux.

Cette caractéristique, en plus de participer à augmenter l'efficacité de la stérilisation des premier mors et second mors, permet une fixation et une désolidarisation rapides desdits mors.

Dans des modes particuliers de réalisation de l'invention, le système de navigation optique est doté d'éléments optiques de référence mécaniquement liés à chacun des premier mors et second mors et d'un module de lecture déterminant la position de chaque élément optique de référence, et l'unité de contrôle est configurée pour déterminer, sur la base d'informations relatives à la position des éléments optiques transmises par le système de navigation optique, la position desdits premier mors et second mors.

Grace à ces caractéristiques, il est possible de connaître de manière automatique la position des mors au sein du porte-outil.

Dans des modes particuliers de réalisation de l'invention, le dispositif de guidage comporte un capteur logé dans une des gorges et configuré pour déterminer la longueur de la course d'une aiguille à travers le conduit de guidage lorsque les mors sont en position de guidage.

Le capteur peut avantageusement être connecté à l'unité de contrôle qui est configurée pour déterminer, sur la base des informations relatives à la longueur de la course d'une aiguille insérée dans le conduit de guidage et sur la base de la position d'une zone anatomique cible d'un patient par rapport à la position du guide aiguille, la position de ladite aiguille par rapport à ladite zone anatomique cible du patient.

Entre également dans le cadre de la présente invention, un dispositif de guidage d'une aiguille en combinaison avec tout ou partie des caractéristiques mentionnées ci-dessus ou ci-après, dans lequel le guide aiguille comporte un organe de transmission de mouvement lié aux mors et synchronisant le déplacement angulaire des mors l'un par rapport à l'autre.

Grâce à cette caractéristique, les deux mors sont animés d'un mouvement angulaire symétrique l'un de l'autre.

En d'autres termes, lors de la rotation d'un mors, l'organe de transmission entraîne la rotation de l'autre mors selon un déplacement angulaire identique.

Ainsi une aiguille peut être enserrée par les gorges sans modifier la position de son axe longitudinal, ce qui permet, par exemple lors d'un changement d'aiguille, d'éviter toute erreur de positionnement de l'aiguille.

En outre, cette caractéristique permet de pouvoir utiliser des aiguilles de différents diamètres.

Dans des modes particuliers de réalisation de l'invention, le dispositif de guidage comporte un organe élastique agencé contre au moins un des mors de sorte à solliciter les premier mors et second mors en rotation vers leur position de guidage.

Cette disposition est avantageuse en ce qu'elle permet d'éviter tout déplacement du guide aiguille dans la position de dégagement, et donc tout déplacement accidentel de l'aiguille.

En outre, cette caractéristique permet d'entraîner de façon systématique les mors en position de guidage, et donc de dispenser un opérateur de réaliser cette opération manuellement.

Dans des modes particuliers de réalisation de l'invention, chaque mors comporte au moins une dent au niveau des gorges, lesdites au moins une dent étant agencées de sorte à s'interpénétrer lorsque le guide aiguille est en position de guidage.

Plus particulièrement, chaque mors comporte au moins une dent, lesdites dents étant agencées en vis-à-vis. Les gorges sont formées transversalement dans les dents respectives des mors.

Ces caractéristiques permettent de répartir les efforts de serrage appliqués sur l'aiguille par les mors, en particulier par les dents, le long de ladite aiguille, et ainsi de participer à garantir la stabilité de l'aiguille lorsqu'elle est engagée dans le conduit de guidage.

Dans des modes particuliers de réalisation de l'invention, les gorges présentent une section transversale en V.

Ainsi, une aiguille est enserrée au sein du conduit de guidage, entre les deux mors, de manière identique quel que soit son diamètre, pour une position de porte-outil donnée ; la position de l'axe longitudinal de ladite aiguille ne dépendant pas de son diamètre. Ainsi il est possible de changer d'aiguille au cours d'une opération en conservant un même axe de translation de l'aiguille, quel que soit son diamètre.

Dans des modes particuliers de réalisation de l'invention, le dispositif de guidage d'une aiguille comprend un mécanisme de verrouillage du guide aiguille en position de guidage. Le mécanisme de verrouillage est configuré pour bloquer en rotation le premier mors ou le second mors lorsque ce dernier pivote au-delà d'une position angulaire prédéterminée.

Cette caractéristique permet avantageusement d'assurer le maintien de l'aiguille par les mors, et plus particulièrement d'interdire tout débattement angulaire de l'aiguille tout en autorisant une translation suivant son axe longitudinal.

Ainsi, toute sollicitation transversale accidentelle de l'aiguille ne peut entraîner un déplacement l'aiguille qui serait susceptible de désengager l'aiguille du conduit de guidage et finalement de blesser un patient.

Dans des modes particuliers de réalisation de l'invention, le mécanisme de verrouillage comporte :
- une liaison pivot reliant la poignée de manipulation au premier mors ou au second mors, et autorisant un degré de liberté en rotation de ladite poignée par rapport audit mors entre deux positions angulaires extrêmes,
- une lèvre s'étendant depuis la poignée de manipulation vers le porte-outil et
- un organe élastique sollicitant ladite poignée en rotation vers l'une de ses positions angulaires extrêmes de sorte que la lèvre coopère avec une surface de contact du porte-outil par arc-boutement lorsque les premier mors et second mors sont en position de guidage.

Autrement dit, l'organe élastique sollicite la poignée de manipulation de sorte à générer le phénomène d'arc-boutement de la lèvre contre la surface de contact du porte-outil.

Cette caractéristique permet de bloquer du guide aiguille en position de guidage par des moyens mécaniques simples.

Un autre avantage réside dans la rapidité de déblocage du guide aiguille pour autoriser son déplacement en position de dégagement.

En effet, il est seulement nécessaire d'appliquer une force allant à l'encontre de la sollicitation de l'organe élastique, sur la poignée de manipulation, pour réduire et/ou supprimer les frottements à l'origine du phénomène d'arc-boutement.

Selon un autre aspect, la présente invention concerne également un bras robotisé comportant à l'une de ses extrémités un dispositif de guidage d'une aiguille tel que précédemment décrit.

Le bras robotisé comprend une unité de contrôle destinée à recevoir des informations relatives à une position du premier mors ou du second mors d'un système de navigation optique du dispositif de guidage d'aiguille. L'unité de contrôle étant configurée pour déterminer la position du mors mobile et pour commander la configuration du bras robotisé dans une position donnée selon la position déterminée dudit mors.

Dans des modes particuliers de réalisation de l'invention, l'unité de contrôle est configurée de sorte que :
- lorsqu'elle détermine que les premier mors et second mors sont en position de guidage, elle interdit tout déplacement du bras robotisé et
- lorsqu'elle détermine que les premier mors et second mors sont en position de dégagement, elle autorise le déplacement du bras robotisé.

Il n'est donc pas possible de modifier la position du dispositif de guidage, et donc de l'axe de translation d'une aiguille insérée dans le conduit de guidage.

Cette caractéristique permet en outre de conserver la position du conduit de guidage lors d'une opération médicale de sorte à garantir la précision de l'insertion de l'aiguille dans une zone anatomique cible du patient.

En outre, cette caractéristique permet d'éviter toute blessure que provoquerait tout déplacement accidentel du bras robotisé portant un guide aiguille dans lequel est insérée une aiguille.

Lorsque les premier mors et second mors sont en position de dégagement, la mobilité du bras robotisé est autorisée de sorte qu'il peut être déplacé latéralement à la zone anatomique cible du patient afin de dégager l'aiguille du guide aiguille sans qu'elle n'ait de contact avec le dispositif de guidage. L'aiguille reste ainsi immobile lors de son dégagement.

### Brève description des figures

L'invention sera mieux comprise à la lecture de la description suivante, donnée à titre d'exemple nullement limitatif, et faite en se référant aux figures suivantes :
[Fig. 1] la figure 1 représente une vue en perspective éclatée d'un bras robotisé, d'un dispositif de guidage d'une aiguille selon une première forme de réalisation et d'un changeur d'outil faisant l'interface entre l'extrémité libre du bras robotisé et le dispositif de guidage ;
[Fig. 2] la figure 2 représente une vue de dessus en perspective du dispositif de guidage de la figure 1, le dispositif de guidage comportant un porte-outil dont seulement une portion est représentée et un guide aiguille qui est illustré dans une position de guidage d'une aiguille ;
[Fig. 3] la figure 3 représente une vue de dessous en perspective du dispositif de guidage de la figure 2 ;
[Fig. 4] la figure 4 représente une vue de dessus en perspective du guide aiguille du dispositif de guidage de la figure 2 ;
[Fig. 5] la figure 5 représente une vue de dessus en perspective de dessus d'un mors mobile du guide aiguille de la figure 2 ;
[Fig. 6] la figure 6 représente une vue en perspective de dessous d'un mors fixe du guide aiguille de la figure 2
[Fig. 7] la figure 7 représente une vue en perspective d'une seconde forme de réalisation d'un dispositif de guidage d'une aiguille
[Fig. 8] la figure 8 représente une vue en coupe du dispositif de guidage d'une aiguille de la figure 7
[Fig. 9] la figure 9 représente une vue en perspective d'une coupe transversale du dispositif de la figure 7.

Dans ces figures, des références numériques identiques d'une figure à l'autre désignent des éléments identiques ou analogues. Par ailleurs, pour des raisons de clarté, les dessins ne sont pas à l'échelle, sauf mention contraire.

### Description des modes de réalisation

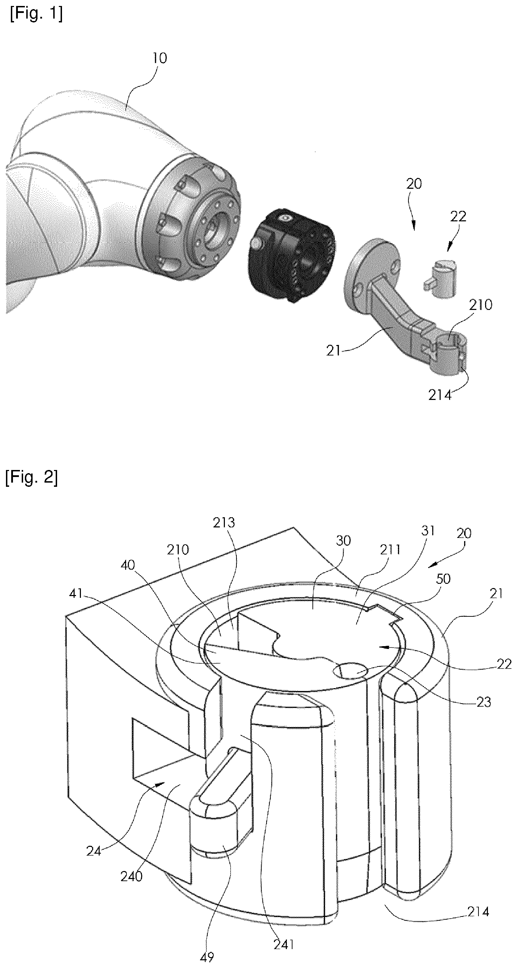

La figure 1 illustre un bras robotisé 10 comportant à son extrémité libre un dispositif de guidage 20 d'une aiguille selon une première forme de réalisation, destiné à assister un opérateur lors d'une opération médicale pour l'introduction d'une aiguille dans le corps d'un patient jusqu'à son insertion dans une zone anatomique cible. Préférentiellement, le bras robotisé 10 comporte un changeur d'outil à son extrémité libre réalisant l'interface entre ladite extrémité libre et le dispositif de guidage 20.

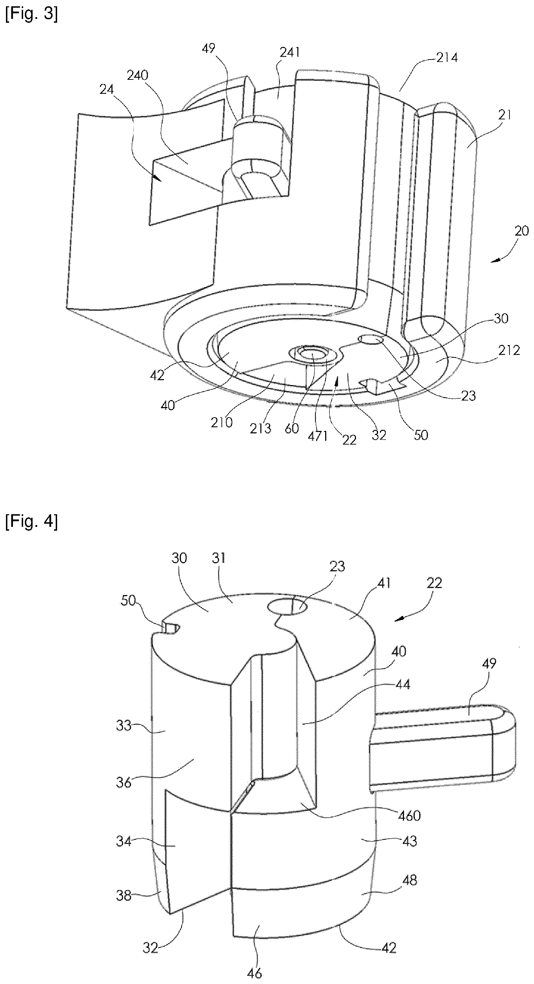

Le dispositif de guidage 20 comporte un porte-outil 21 destiné à être fixé au bras robotisé 10, et un guide aiguille 22 coopérant avec ledit porte-outil 21, comme l'illustrent les figures 2 et 3.

Le porte-outil 21 comporte un logement 210 s'étendant longitudinalement entre deux ouvertures d'extrémité débouchant respectivement sur une face dite « face supérieure » 211 et sur une face dite « face inférieure » 212 du porte-outil 21. Le logement 210 est défini, entre ses deux ouvertures d'extrémités, par une paroi interne 213.

La paroi interne 213 présente une section droite de forme circulaire dans l'exemple préféré de réalisation représenté sur les figures 1 à 3.

Le guide aiguille 22 est engagé dans le logement 210 et comporte, dans l'exemple préféré de réalisation de l'invention, deux mors 30, 40 fixés l'un à l'autre de manière libre en rotation, entre une « position de guidage » dans laquelle lesdits mors 30, 40 définissent un conduit de guidage 23 d'une aiguille et une « position de dégagement » dans laquelle ils définissent une zone de dégagement latéral d'une aiguille.

Les mors sont appelés ci-après de façon générale « premier mors » 30 et « second mors » 40.

Comme l'illustrent les figures 1 à 3, le porte-outil 21 comporte une ouverture axiale traversante 214 s'étendant de l'une à l'autre des ouvertures d'extrémité du logement 210 selon un axe parallèle à l'axe longitudinal du logement 210. Avantageusement, les premier mors 30 et second mors 40 sont disposés dans le logement 210 de sorte que, lorsqu'ils sont en position de dégagement, la zone de dégagement latéral est en regard de l'ouverture axiale traversante 214.

Ainsi, une aiguille insérée dans le conduit de guidage 23 lorsque les premier mors 30 et second mors 40 sont en position de guidage, en vue de son introduction dans une zone anatomique cible d'un patient, peut être retirée latéralement du dispositif de guidage 20 lorsque les premier mors 30 et second mors 40 occupent la position de dégagement, à travers la zone de dégagement latéral et l'ouverture axiale traversante 214, par déplacement dudit dispositif de guidage 20.

Préférentiellement, dans une première forme de réalisation, lorsque le guide aiguille 22 est engagé dans le logement 210, le premier mors 30, dit « mors fixe » 30, est immobilisé par rapport au porte-outil 21, et le second mors 40, dit « mors mobile » 40 est libre en rotation par rapport audit mors fixe 30 selon un axe de rotation parallèle à l'axe longitudinal du logement 210.

Plus particulièrement, comme le montrent les figures 4 à 6, le mors fixe 30 et le mors mobile 40 s'étendent entre deux extrémités, respectivement appelées « extrémité supérieure » 31, 41 et « extrémité inférieure » 32, 42, selon des axes longitudinaux parallèles à l'axe longitudinal du logement 210. Lesdits mors fixe 30 et mors mobile 40 sont respectivement définis, entre leurs extrémités supérieure 31 et 41 et inférieure 32 et 42, par une surface périphérique dont une portion appelée ci-après « surface extrados » 33, 43 est reliée à une portion appelée ci-après « surface intrados » 34, 44 à laquelle elle est opposée.

Il y a lieu de noter que, dans le présent texte, les termes relatifs « supérieur » et « inférieur » sont définis de sorte qu'un élément dit « supérieur » est situé au-dessus d'un élément dit « inférieur », lesdits termes relatifs se rattachant à la position dans laquelle le bras robotisé 10 et le dispositif de guidage 20 sont représentés sur les figures 1 à 3.

La surface extrados 33, 43 de chacun des mors fixe 30 et mors mobile 40 présente une section droite de forme circulaire et est agencé en regard de la paroi interne 213 du logement 210 lorsque le guide aiguille 22 est engagé dans le porte-outil 21.

Comme le montre la figure 4 dans l'exemple préféré de réalisation de l'invention, les surfaces extrados 33, 43 des mors fixe 30 et mors mobile 40 sont inscrites dans un cylindre de révolution de section droite circulaire. Les surfaces extrados 33, 43 et la paroi interne 213 du logement 210 sont concentriques lorsque lesdits mors fixe 30 et mors mobile 40 sont engagés dans le logement 210.

Afin d'être engagés dans le logement 210, le mors fixe 30 et le mors mobile 40 sont dimensionnés de sorte que le rayon de la section droite de leur surface extrados 33, 43 est plus faible que le rayon de la section droite de la paroi interne 213 dudit logement 210.

Les surfaces intrados 34, 44 des mors fixe 30 et mors mobile 40 sont agencées en vis-à-vis l'une de l'autre lorsque le guide aiguille 22 est engagé dans le porte-outil 21 et sont dimensionnées de sorte à ménager un espace de débattement angulaire entre lesdits mors fixe 30 et mors mobile 40.

Chaque mors fixe 30 et mors mobile 40 comprend, sur sa surface intrados 34, 44, une gorge 35, 45 s'étendant selon un axe longitudinal, depuis son extrémité inférieure 32, 42 jusqu'à son extrémité supérieure 31, 41. Les gorges 35, 45 présentent respectivement une section droite de forme circulaire de dimensions identiques l'une de l'autre. Alternativement, les gorges 35, 45 peuvent présenter une section droite de forme polygonale.

Les gorges 35, 45 sont agencées de sorte que lorsque le mors fixe 30 et le mors mobile 40 occupent la position de guidage, lesdites gorges 35, 45 sont attenantes l'une à l'autre sur l'ensemble de leur longueur et forment le conduit de guidage 23 d'une aiguille et de sorte que lorsque les mors occupent la position de dégagement, lesdites gorges 35, 45 sont éloignées l'une de l'autre et définissent entre elles la zone de dégagement latéral d'une aiguille.

Préférentiellement, les gorges 35, 45 sont respectivement agencées sur une portion de la surface intrados 34, 44 de chacun des mors fixe 30 et mors mobile 40 comprise dans un plan diamétral à la surface extrados 33, 43.

Dans l'exemple préféré de réalisation de l'invention, le mors fixe 30 et le logement 210 présentent, sur leurs surfaces respectives en vis-à-vis, c'est-à-dire la surface extrados 33 du mors fixe 30 et la paroi interne 213 du logement 210, des éléments d'emboitement 50 coopérant l'un avec l'autre pour immobiliser ledit mors fixe 30 en rotation par rapport au porte-outil 21.

Avantageusement, les éléments d'emboitement 50 peuvent autoriser un degré de liberté en translation entre le porte-outil 21 et le mors fixe 30 de sorte que le guide aiguille 22 puisse être engagé dans le logement 210 du porte-outil 21 de manière amovible comme décrit plus en détail ci-après.

Préférentiellement, les éléments d'emboitement 50 sont formés par des reliefs de formes complémentaires s'étendant respectivement parallèlement à l'axe longitudinal du mors fixe 30 et à l'axe longitudinal du logement 210. Ces reliefs présentent respectivement, par exemple, la forme d'une languette et d'une rainure, la languette étant, dans la forme préférée de réalisation, agencée sur la surface extrados 33 du mors fixe 30 et la rainure étant agencée sur la paroi interne 213 du logement 210, comme le montre la figure 2. Alternativement, la languette est agencée sur la paroi interne 213 du logement 210 et la rainure est agencée sur la surface extrados 33 du mors fixe 30.

Ainsi, l'immobilisation du mors fixe 30 est assurée de façon simple et fiable.

Les éléments d'emboitement 50 peuvent être alternativement formés par des rainures agencées longitudinalement en vis-à-vis l'une de l'autre, respectivement sur la surface extrados 33 du mors fixe 30 et sur la paroi interne 213 du logement 210, comme illustré sur la figure 3, et dans lesquelles est destinée à être engagée une clavette ou une goupille.

Les éléments d'emboitement 50 présentent comme avantage de jouer un rôle de détrompeur lors de l'insertion du guide aiguille 22 dans le logement 210.

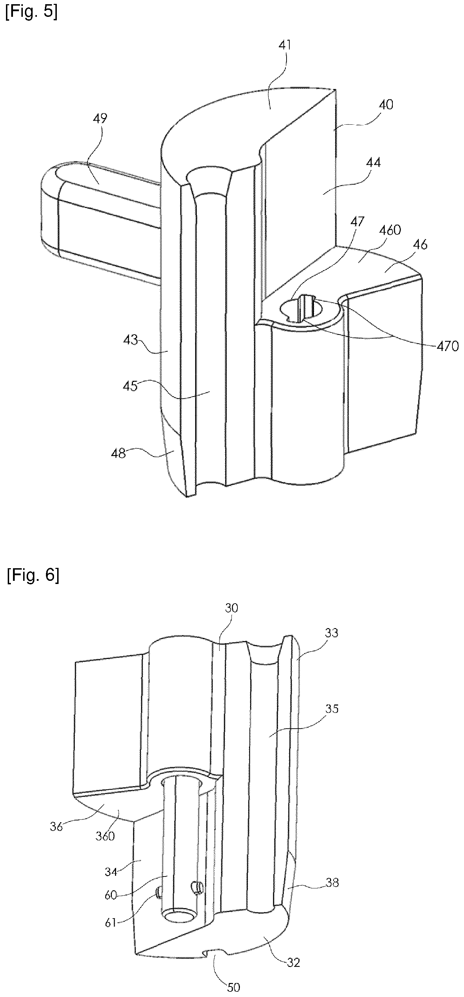

Comme le montrent les vues isolées des mors fixe 30 et mors mobile 40 des figures 5 et 6, chacun desdits mors fixe 30 et mors mobile 40 présente un épaulement axial 36, 46 sur sa surface intrados 34, 44, par lequel lesdits mors coopèrent l'un avec l'autre.

Les épaulements axiaux 36, 46 de chacun des mors fixe 30 et mors mobile 40 s'étendent de manière opposée l'un de l'autre depuis une surface d'appui 360, 460 comprise dans un plan sensiblement orthogonal à l'axe longitudinal desdits mors, jusqu'à une surface affleurant l'une ou l'autre des extrémités supérieure 31, 41 et inférieure 32, 42 desdits mors fixe 30 et mors mobile 40.

Plus précisément, comme représenté par les figures 4 et 6, l'épaulement axial 36 du mors fixe 30 s'étend jusqu'à une surface affleurant son extrémité supérieure 31 et l'épaulement axial 46 du mors mobile 40 s'étend jusqu'à une surface affleurant son extrémité inférieure 42. Autrement dit, l'épaulement axial 36 du mors fixe 30 est superposé à celui du mors mobile 40.

Dans l'exemple préféré de réalisation de l'invention, la surface d'appui 360, 460 de l'épaulement axial 36, 46 de chacun des mors fixe 30 et mors mobile 40 est agencée à équidistance entre les extrémités supérieure 31, 41 et inférieure 32, 42 desdits mors.

Les épaulements axiaux 36, 46 reposent l'un contre l'autre par leurs surfaces d'appui 360, 460 respectives et présentent des profils complémentaires de sorte que le mors fixe 30 et le mors mobile 40 sont imbriqués au moins partiellement l'une avec l'autre.

Les épaulements axiaux 36, 46 comportent une portion centrale par laquelle le mors fixe 30 et le mors mobile 40 reposent en appui l'un contre l'autre de manière continue lorsqu'ils évoluent entre leurs positions de dégagement et de guidage, comme l'illustre la figure 4.

Comme le montrent les figures 5 et 6, la section droite de la surface intrados 34, 44, au niveau de la portion centrale de chaque épaulement axial 36, 46, présente un segment de forme circulaire, ledit segment de forme circulaire étant concentrique avec la section droite de la surface extrados 33, 43 et celle de la paroi interne 213.

Les épaulements axiaux 36, 46 présentent également respectivement une portion latérale prolongeant la portion centrale jusqu'à une surface cylindrique s'étendant dans le prolongement de la surface extrados 33, 43 des mors fixe 30 et mors mobile 40. Autrement dit, la surface cylindrique est inscrite dans un même cylindre de révolution que les surfaces extrados 33, 43 des mors fixe 30 et mors mobile 40.

La portion latérale a donc une forme générale d'un secteur de cylindre.

Comme le montrent les figures 5 et 6, la section droite de la surface intrados 34, 44 présente, au niveau de la portion latérale de chaque épaulement axial 36, 46, un segment rectiligne prolongeant le segment de forme circulaire jusqu'à la surface extrados 33, 43.

Les portions latérales des épaulements axiaux 36, 46 sont configurées de sorte à constituer une butée de débattement angulaire du mors mobile 40 lorsque le mors mobile 40 est en position de dégagement.

Plus particulièrement, les portions latérales sont dimensionnées de sorte que, lorsque le mors mobile 40 est en position de dégagement, elles reposent en appui l'une contre l'autre par leur surface d'appui 360, 460, la portion latérale de l'épaulement axial 36 du mors fixe 30 venant au contact de la surface intrados 44 du mors mobile 40 et la portion latérale de l'épaulement axial 46 du mors mobile 40 venant au contact de la surface intrados 34 du mors fixe 30.

Préférentiellement, les portions latérales des épaulements axiaux 36, 46 sont dimensionnées de sorte à permettre un débattement angulaire du mors mobile 40 de quarante-cinq degrés entre les positions de guidage et de dégagement.

Cette caractéristique permet de maximiser la surface de contact entre le mors fixe 30 et le mors mobile 40, et donc de bénéficier d'une précision importante dans le déplacement du mors mobile 40 lorsqu'elle évolue entre les positions de dégagement et de guidage.

Le mors fixe 30 et le mors mobile 40 sont préférentiellement liés entre eux de manière amovible par l'intermédiaire d'une tige 60 s'étendant longitudinalement depuis le mors fixe à travers un logement s'étendant axialement, dit « logement axial » 47, formé dans l'épaulement axial 46 du mors mobile 40, ledit mors mobile 40 étant libre de pivoter autour de la tige 60. Autrement dit, la tige 60 s'étend dans le logement axial 47 selon un axe longitudinal confondu avec l'axe de rotation du mors mobile 40.

Plus particulièrement, comme le montrent les figures 5 et 6, le logement axial 47 est formé à travers la portion centrale de l'épaulement axial 46 du mors mobile 40.

La tige 60 s'étend entre deux extrémités, dont une dite « extrémité supérieure » est rigidement liée au mors fixe 30 et dont l'autre dite « extrémité inférieure » est engagée de manière libre en rotation dans le logement axial 47.

Grâce à cette caractéristique, le mors fixe 30 et le mors mobile 40 peuvent être désolidarisés l'une de l'autre. Une telle disposition vise notamment à augmenter l'efficacité de la stérilisation du guide aiguille 22.

L'extrémité inférieure de la tige 60 comporte avantageusement un élément de blocage en translation adapté à interdire toute translation entre le mors fixe 30 et le mors mobile 40. Cette caractéristique vise à éviter toute désolidarisation intempestive des mors fixe 30 et mors mobile 40.

Un tel élément de blocage en translation peut être formé par une goupille 61 s'étendant radialement dans ladite tige 60 comme le montre la figure 6. La goupille 61 est destinée à être agencée contre le mors mobile 40 de sorte à maintenir ledit mors mobile 40 contre le mors fixe 30.

Plus précisément, comme le montre la figure 3, le mors mobile 40 présente avantageusement un chambrage 471 par lequel le logement axial 47 débouche sur son extrémité inférieure 42, ledit chambrage 471 est prévu pour recevoir la goupille 61 lorsque le mors fixe 30 est fixé au mors mobile 40.

La figure 5 illustre deux rainures axiales 470 diamétralement opposées et s'étendant sur l'ensemble de la longueur du logement axial 47. Ces rainures axiales 470 sont destinées à recevoir la goupille 61 en coulissement lors de la fixation ou lors de la désolidarisation des mors fixe 30 et mors mobile 40 l'un avec l'autre. Alternativement, suivant la longueur de la goupille 61, le logement axial 47 du mors mobile 40 peut ne comprendre qu'une unique rainure axiale 470.

Préférentiellement, les rainures axiales 470 sont agencées au sein du logement axial 47 de sorte que la fixation ou la désolidarisation des mors fixe 30 et mors mobile 40 l'un avec l'autre est possible seulement lorsque lesdits mors sont positionnés angulairement l'un par rapport à l'autre à équidistance entre la position de dégagement et la position de guidage, c'est-à-dire à mi-course entre les deux positions extrêmes du mors mobile 40.

Avantageusement, le mors mobile 40 comporte une poignée de manipulation 49 s'étendant radialement depuis sa surface extrados 43. La poignée de manipulation 49 permet d'entraîner en déplacement audit mors mobile 40 au sein du logement 210 sous la sollicitation d'un opérateur. Cette poignée de manipulation 49 est prévue pour évoluer à travers une lumière 24 formée dans le porte-outil 21 et s'étendant radialement par rapport au logement 210 par une portion appelée ci-après « portion radiale » 240.

La lumière 24 forme ainsi un chemin de guidage de la poignée de manipulation 49 et s'étend selon une longueur suffisante pour permettre un débattement angulaire du mors mobile 40 entre les positions de dégagement et de guidage.

Comme illustré sur les figures 2 et 3, la portion radiale 240 est prolongée, préférentiellement à l'une de ses extrémités, par une portion, dite « portion axiale » 241, s'étendant axialement par rapport au logement 210, jusqu'à déboucher sur la face supérieure 211 du porte-outil 21.

Une telle caractéristique est avantageuse car elle permet, par un pivotement de la poignée de manipulation 49 dans la portion radiale 240 de la lumière 24 suivi d'une translation de ladite poignée dans la portion axiale 241 selon l'axe longitudinal du logement 210, de désolidariser le guide aiguille 22 du porte-outil 21. Ainsi, la stérilisation du porte-outil 21 et du guide aiguille 22 peut être réalisée de façon optimale.

A l'inverse, pour engager le guide aiguille 22 dans le porte-outil 21, un opérateur introduit la poignée de manipulation 49 à travers la portion axiale 241 de la lumière 24, par une translation du guide aiguille 22 selon l'axe longitudinal du logement 210, jusqu'à ce qu'elle atteigne la portion radiale 240.

Avantageusement, la portion axiale est agencée de sorte que la désolidarisation du guide aiguille 22 et du porte-outil 21 soit autorisée seulement lorsque le mors mobile 40 est en position de guidage.

Il est ainsi possible de guider une aiguille axialement directement suite à l'installation du guide aiguille 22 au sein du logement 210.

Il est ainsi évité tout risque de coincement de l'aiguille en dehors du conduit de guidage 23 entre le mors fixe 30 et le mors mobile 40, lors d'un actionnement dudit mors mobile 40 vers sa position de guidage ; un tel coincement pouvant occasionner un guidage de l'aiguille incertain et potentiellement dangereux pour un patient.

Avantageusement, afin de faciliter l'engagement du guide aiguille 22 dans le logement 210, le mors fixe 30 et le mors mobile 40 peuvent comprendre un chanfrein 38, 48 s'étendant entre leur extrémité inférieure 32, 42 et leur surface extrados 33, 43 respectives.

Dans une variante de la première forme de réalisation du guide aiguille 20 non représenté sur les figures, le porte-outil 21 et le mors fixe forment une pièce d'un seul tenant. Dans cet exemple de réalisation de l'invention, le mors mobile correspond à la description qui en est faite ci-dessus, à la différence près que son épaulement axial s'étend depuis une surface d'appui jusqu'à une surface affleurant l'extrémité supérieure dudit mors mobile.

Dans cet exemple de réalisation de l'invention, l'épaulement axial du mors fixe s'étend depuis une surface d'appui jusqu'à une surface affleurant l'extrémité inférieure dudit mors fixe.

On comprend ici que l'épaulement axial du mors mobile est superposé à celui du mors fixe, c'est-à-dire celui du porte-outil 21.

En outre, la tige est rigidement fixée au mors mobile et est engagée de manière libre en rotation dans le logement axial du mors fixe. Ledit logement axial présente deux rainures axiales diamétralement opposées s'étendant depuis la surface d'appui de l'épaulement axial jusqu'à un chambrage débouchant sur la face inférieure dudit porte-outil 21. De manière analogue à l'exemple préféré de réalisation de l'invention, la tige présente à son extrémité inférieure un élément de blocage en translation, tel qu'une goupille, que les rainures sont destinées à recevoir en coulissement lors de la fixation ou la désolidarisation du mors mobile du porte-outil 21.

Dans cet exemple de réalisation de l'invention, le porte-outil 21 comprend un capteur agencé de sorte à déterminer la longueur de la course d'une aiguille évoluant à travers le conduit de guidage lorsque les mors sont en position de guidage.

Avantageusement, le capteur est connecté à l'unité de contrôle qui détermine, sur la base des informations relatives à la longueur de la course d'une aiguille insérée dans le conduit de guidage et sur la base de la position d'une zone anatomique cible d'un patient par rapport à la position du guide aiguille, la position de ladite aiguille par rapport à ladite zone anatomique cible du patient.

Le capteur peut être un capteur optique connu en tant que tel par l'homme du métier, agencé de sorte à détecter l'évolution de l'aiguille introduite à travers le conduit de guidage en capturant des images. Le capteur peut également être formé par un capteur de mesure linéaire également connu en tant que tel par l'homme du métier.

Alternativement, le porte-outil 21 peut intégrer un capteur de type codeur optique linéaire adapté à déterminer la course d'une aiguille graduée évoluant dans le conduit de guidage par la lecture des graduations de ladite aiguille.

Le capteur peut, encore alternativement, être formé par une roulette entraînée par l'aiguille lors de son insertion à travers le conduit, ladite roulette étant associée à un compte-tour, tel qu'un tachymètre. Le diamètre de la roulette étant connu par l'unité de contrôle, ladite unité de contrôle peut déterminer la longueur d'aiguille insérée à travers le conduit de guidage en fonction du déplacement angulaire réalisé au cours du déplacement de l'aiguille.

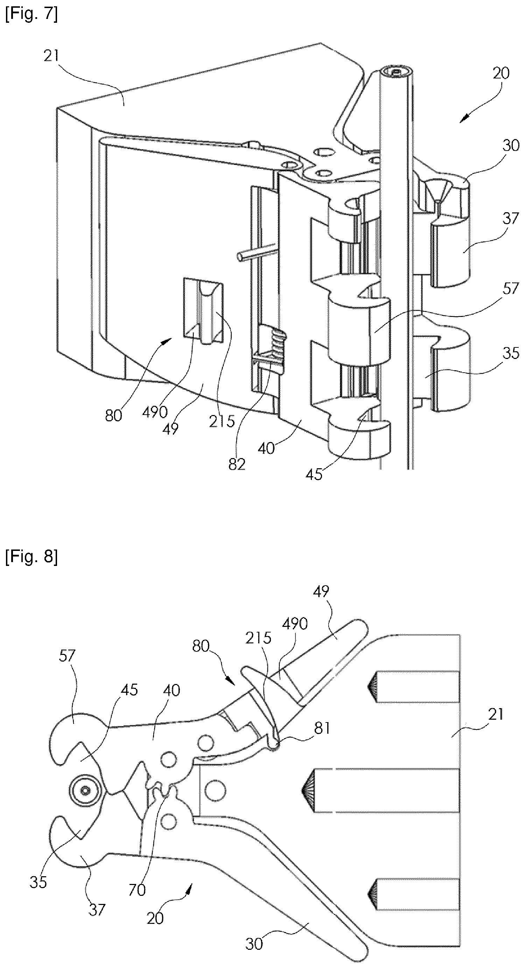

Les figures 7 à 9 représentent un dispositif de guidage 20 d'une aiguille selon une seconde forme de réalisation.

Le dispositif de guidage 20 selon cette seconde forme de réalisation est conforme au dispositif de guidage 20 selon la première forme de réalisation en ce qu'il présente également un porte-outil 21, un guide aiguille 22 comportant un premier et un second mors 30 et 40 mobiles l'un par rapport à l'autre entre une position de guidage et une position de dégagement.

En outre, dans cette seconde forme de réalisation, le second mors 40, appelé ici « mors menant » 40, comporte également une poignée de manipulation 49. Cependant, le porte-outil 21 ne présente pas de lumière dans laquelle évolue la poignée de manipulation 49.

Le premier mors 30 est appelé dans cette forme de réalisation « mors mené » 30.

Contrairement à la première forme de réalisation, dans cette seconde forme de réalisation, le mors mené 30 et le mors menant 40 sont fixés de manière mobile en rotation au porte-outil 21 selon des axes de rotation respectifs distincts, c'est-à-dire non coaxiaux. Les axes de rotation du mors mené 30 et du mors menant 40 sont préférentiellement parllèles entre eux.

Le porte-outil 21 comprend une chappe comportant deux oreilles dont une « oreille supérieure » et une « oreille inférieure » entre lesquelles sont agencées le mors mené 30 et le mors menant 40. Seule l'oreille supérieure est visible sur la figure 7.

Les oreilles supérieure et inférieure présentent une paire d'orifices, les orifices des deux paires en vis-à-vis recevant une broche de fixation, chaque broche de fixation étant engagée dans un logement traversant d'un des mors mené 30 et mors menant 40.

Ainsi, chaque mors mené 30 et menant 40 est libre de pivoter autour de la broche avec laquelle il est associé.

Comme le montrent les figures 7 à 9, le guide aiguille comporte avantageusement un organe de transmission de mouvement 70 lié aux mors mené 30 et mors menant 40, synchronisant le déplacement angulaire desdits mors mené 30 et mors menant 40 l'un par rapport à l'autre.

Préférentiellement, comme illustré sur la figure 8, l'organe de transmission de mouvement 70 est formé par deux portions de roues dentées respectivement agencées sur chacun des mors mené 30 et mors menant 40 et en relation d'engrènement l'une avec l'autre.

Alternativement, l'organe de transmission de mouvement 70 peut être formé par un mécanisme de came (non représenté sur les figures) dans lequel une partie mâle s'étendant depuis l'un des mors, par exemple le mors menant 40 coopère avec une partie femelle s'étendant depuis l'autre mors, par exemple le mors mené 30.

Grâce à cette caractéristique, le mors mené 30 et le mors menant 40 sont animés d'un mouvement angulaire symétrique l'un de l'autre, c'est-à-dire qu'ils se déplacent selon un même angle.

Ainsi une aiguille peut être enserrée par les gorges 35, 45 sans modifier la position de son axe longitudinal, ce qui permet, par exemple lors d'un changement d'aiguille, d'éviter toute erreur de positionnement de l'aiguille.

En outre, cette caractéristique permet de pouvoir utiliser des aiguilles de différents diamètres. Préférentiellement, cette caractéristique permet de guider des aiguilles dont le diamètre est compris entre 11G et 21G.

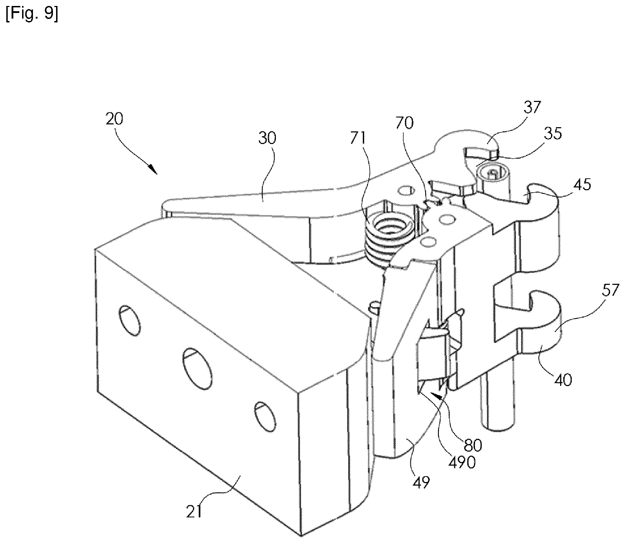

Un organe élastique 71 est agencé contre au moins un des mors mené 30 ou menant 40 de sorte à solliciter lesdits mors mené 30 et mors menant 40 en rotation vers leur position de guidage, comme le représente la figure 9.

Plus particulièrement, l'organe élastique 71 peut être un ressort de torsion agencé entre le mors mené 30 et le mors menant 40, à l'opposé de leur gorge 35, 45, et dont chacune des extrémités est en appui contre l'un desdits mors mené 30 et mors menant 40. Alternativement, l'organe élastique 71 peut être un ressort de compression.

Cette disposition est avantageuse en ce qu'elle permet d'éviter tout déplacement du guide aiguille 22 dans la position de dégagement, et donc tout déplacement accidentel de l'aiguille.

En outre, cette caractéristique permet d'entraîner de façon systématique le mors mené 30 et le mors menant 40 en position de guidage, et donc de dispenser un opérateur de réaliser cette opération manuellement.

Avantageusement, le mors mené 30 et le mors menant 40 comportent, à la jonction entre leur surface intrados 34, 44 et leur surface extrados 33, 43, des dents 37, 57 agencées de sorte à s'interpénétrer lorsque le guide aiguille 22 est en position de guidage.

Ainsi, comme illustré sur les figures 7 à 9, chaque dent 37, 57 présente un segment d'une gorge 35, 45, lesdites gorges 35, 45 s'étendant transversalement par rapport aux dents 37, 57.

Ces caractéristiques permettent de répartir les efforts de serrage appliqués sur l'aiguille par les mors mené 30 et menant 40, en particulier par les dents 37, 57, le long de ladite aiguille, et ainsi de participer à garantir la stabilité de l'aiguille lorsqu'elle est engagée dans le conduit de guidage 23.

Préférentiellement, les gorges 35, 45 présentent une section transversale en V.

Ainsi, une aiguille est enserrée au sein du conduit de guidage 23, entre les mors mené 30 et mors menant 40, dans une position identique quel que soit son diamètre, pour une position de porte-outil 21 donnée ; en d'autres termes, la position de l'axe longitudinal de ladite aiguille ne dépend pas de son diamètre. Ainsi il est possible de changer d'aiguille au cours d'une opération en conservant un même axe de translation de l'aiguille, quel que soit son diamètre.

Sur les figures 7 et 8, le dispositif de guidage 20 est représenté avec un mécanisme de verrouillage 80 du guide aiguille 22 en position de guidage.

Le mécanisme de verrouillage 80 est configuré pour bloquer en rotation l'un des mors, préférentiellement le mors menant 40, lorsque ce dernier pivote au-delà d'une position angulaire prédéterminée. La position angulaire prédéterminée correspond à la position des mors lorsqu'ils sont en position de guidage.

L'immobilisation en rotation du mors menant 40 permet par extension d'interdire le pivotement du mors mené 30 grâce à l'organe de transmission assujétissant les mors l'un à l'autre.

Le mécanisme de verrouillage 80 permet avantageusement d'assurer le maintien de l'aiguille par le mors mené 30 et le mors menant 40, et plus particulièrement d'interdire tout débattement angulaire de l'aiguille tout en autorisant une translation suivant son axe longitudinal.

Ainsi, toute sollicitation transversale accidentelle de l'aiguille ne peut entraîner un déplacement l'aiguille qui serait susceptible de désengager l'aiguille du conduit de guidage et finalement de blesser un patient.

Le mécanisme de verrouillage 80 comporte une liaison pivot reliant la poignée de manipulation 49 au mors menant 40. La poignée de manipulation 49 est ainsi libre de pivoter par rapport audit mors menant 40 entre deux positions angulaires extrêmes appelées ci-après « position de blocage » et « position de déblocage ».

La poignée de manipulation 49 présente avantageusement un épaulement configuré pour coopérer avec un épaulement du mors menant 40 de sorte à interdire la rotation de la poignée de manutention par rapport au mors menant 40 lorsqu'elle pivote vers sa position de déblocage, comme visible sur les vues en coupe des figures 8 et 9. Ces épaulements forment une première butée angulaire.

La poignée de manipulation 49 et le mors menant 40 peuvent également être conformés de sorte à coopérer l'un avec l'autre de sorte à interdire la rotation de ladite poignée de manipulation 49 par rapport au mors menant 40 lorsqu'elle pivote vers sa position de blocage, de sorte à former une seconde butée angulaire visible sur la figure 7.

Dans l'exemple de mécanisme de verrouillage représenté sur les figures 7à 9, une lèvre 81 s'étend depuis la poignée de manipulation 49 vers le porte-outil 21.

Lorsque la poignée de manipulation 49 occupe la position de blocage, la lèvre 81 est destinée à reposer en appui contre une surface du porte-outil 21. À cet effet, le porte-outil 21 comporte une surface de contact 215 destinée à recevoir ladite lèvre 81 en appui.

Préférentiellement, la surface de contact 215 présente une section droite sensiblement concave et s'étend par exemple depuis une cavité réalisée dans le porte-outil 21, jusqu'à une excroissance, comme le montrent les figures 7 à 9.

Le porte-outil 21 est configuré de sorte que, lorsque le mors mené 30 et le mors menant 40 sont en position de dégagement, la lèvre 81 est engagée dans la cavité. La cavité est donc dans la prolongation de la course angulaire de la lèvre 81.

Comme le montrent les figures 7 à 9, la poignée de manutention 49 peut comprendre une ouverture 490 à travers laquelle s'introduit l'excroissance du porte-outil lorsque le mors mené 30 et le mors menant 40 évoluent vers leur position de dégagement.

Avantageusement, le mécanisme de verrouillage 80 comporte un organe élastique 82 sollicitant ladite poignée en rotation vers sa position de blocage de sorte que la lèvre 81 coopère avec la surface de contact 215 du porte-outil 21 par arc-boutement lorsque le mors mené 30 et le mors menant 40 sont en position de guidage.

L'organe élastique 82 est préférentiellement formé par un ressort de torsion agencé entre le mors menant 40 et la poignée de manipulation.

Ce mécanisme de verrouillage 80 est avantageusement efficace quel que soit le diamètre de l'aiguille engagée dans le conduit de guidage.

En outre, cette caractéristique permet de bloquer du guide aiguille en position de guidage par des moyens mécaniques simples.

Un autre avantage réside dans la rapidité de déblocage du guide aiguille pour autoriser son déplacement en position de dégagement.

En effet, il est seulement nécessaire d'appliquer une force allant à l'encontre de la sollicitation de l'organe élastique, sur la poignée de manipulation, pour réduire et/ou supprimer les frottements à l'origine du phénomène d'arc-boutement.

Les dispositions décrites ci-après peuvent avantageusement convenir aux deux formes de réalisation du dispositif de guidage 20 selon la présente invention.

Le dispositif de guidage 20 peut avantageusement comporter un système de navigation optique (non représenté sur les figures) destiné à être connecté à une unité de contrôle du bras robotisé 10 déterminant, sur la base d'informations transmises par ledit système de navigation optique, la position des premier mors 30 et second mors 40 l'un par rapport à l'autre.

L'unité de contrôle est configurée pour piloter les déplacements du bras robotisé 10, et donc les déplacements du guide aiguille 22, en fonction de la position des premier mors 30 et second mors 40 l'un par rapport à l'autre.

Le système de navigation optique comporte des éléments optiques de référence, tels que des sphères dont la surface est réfléchissante.

Les sphères sont portées par des branches s'étendant depuis l'extrémité supérieure 31, 41 de chacun des premier mors 30 et second mors 40.

Le système de navigation optique comporte également un module de lecture transmettant à l'unité de contrôle des informations relatives à la position dans l'espace de chaque élément optique de référence.

Plus particulièrement, le premier mors 30 comporte un élément optique de référence individuel et le second mors 40 comporte au moins une paire d'éléments optiques de référence. La paire d'éléments optiques de référence est portée par une paire de branches s'étendant depuis une branche commune par laquelle ils sont fixés au mors mobile 40.

Sur la base des informations reçues par le module de lecture, l'unité de contrôle est apte à déterminer la position de la paire d'éléments optiques de référence par rapport à l'élément optique de référence individuel et d'en déduire la position des premier mors 30 et second mors 40 l'un par rapport à l'autre.

L'unité de contrôle est configurée de sorte que si elle détermine que le premier mors 30 et second mors 40 sont en position de guidage, elle interdit tout déplacement du bras robotisé 10. Il n'est donc pas possible de modifier la position du conduit de guidage 23, et par extension de déplacer l'axe de translation d'une aiguille insérée dans ledit conduit, ni de libérer latéralement l'aiguille du guide.

Cette caractéristique permet de conserver la position du dispositif de guidage 20 et donc du conduit de guidage 23 lors d'une opération médicale de sorte à garantir la précision de l'insertion de l'aiguille dans une zone anatomique cible du patient.

En outre, cette caractéristique permet d'éviter tout accident susceptible d'être provoqué par le déplacement d'une aiguille insérée dans un guide aiguille 22.

L'unité de contrôle est, en outre, configurée de sorte que si elle détermine que le premier mors 30 et second mors 40 sont en position de dégagement, elle autorise le déplacement du bras robotisé 10. Il est alors possible de déplacer ledit bras robotisé 10 latéralement à la zone anatomique cible du patient de sorte à dégager l'aiguille du guide aiguille 22 sans qu'elle n'ait de contact avec le dispositif de guidage 20. Ainsi, l'aiguille reste immobile lors de son dégagement.

Cette caractéristique permet d'éviter tout accident susceptible d'être provoqué par le déplacement d'une aiguille insérée dans une zone anatomique cible.

De manière plus générale, il est à noter que les modes de mise en œuvre et de réalisation de l'invention considérés ci-dessus ont été décrits à titre d'exemples non limitatifs et que d'autres variantes sont par conséquent envisageables. L'invention est définie par les revendications jointes.

## Revendications

1. Dispositif de guidage (20) d'une aiguille comportant un porte-outil (21) destiné à être fixé à l'extrémité d'un bras robotisé (10) d'assistance médicale, ledit porte-outil (21) supportant un guide aiguille (22),
ledit guide aiguille (22) comportant un premier mors (30) et un second mors (40) dotés respectivement d'une gorge (35, 45), lesdites gorges s'étendant l'une et l'autre selon des axes longitudinaux parallèles, lesdits premier mors (30) et second mors (40) étant supportés par le porte-outil de sorte à autoriser une mobilité en rotation desdits premier mors (30) et second mors (40) l'un par rapport à l'autre entre une position dite « position de guidage », dans laquelle les gorges (35, 45) sont attenantes et définissent un conduit de guidage (23) d'une aiguille, et une position dite « position de dégagement » dans laquelle les gorges (35, 45) sont éloignées l'une de l'autre et définissent une zone de dégagement latéral d'une aiguille,
**caractérisé en ce que** le dispositif de guidage (20) comporte un système de navigation optique destiné à être connecté à une unité de contrôle du bras robotisé (10) déterminant, sur la base d'informations transmises par ledit système de navigation optique, la position des premier mors (30) et second mors (40) l'un par rapport à l'autre.

2. Dispositif de guidage (20) d'une aiguille selon la revendication 1 dans lequel le système de navigation optique comporte des éléments optiques de référence.

3. Dispositif de guidage (20) d'une aiguille selon la revendication 2 dans lequel les éléments optiques de référence sont des sphères dont la surface est réfléchissante.

4. Dispositif de guidage (20) d'une aiguille selon la revendication 3 dans lequel les sphères sont portées par des branches s'étendant depuis une extrémité supérieure (31, 41) de chacun des premier mors (30) et second mors (40).

5. Dispositif de guidage (20) d'une aiguille selon l'une des revendications 2 à 4 dans lequel le premier mors (30) comporte un élément optique de référence individuel et le second mors (40) comporte au moins une paire d'éléments optiques de référence.

6. Dispositif de guidage (20) d'une aiguille selon la revendication 5 dans lequel la paire d'éléments optiques de référence est portée par une paire de branches s'étendant depuis une branche commune par laquelle ils sont fixés au second mors (40).

7. Dispositif de guidage (20) d'une aiguille selon l'une des revendications 2 à 6 dans lequel le système de navigation optique comporte un module de lecture destiné à transmettre à l'unité de contrôle des informations relatives à la position dans l'espace de chaque élément optique de référence.

8. Bras robotisé (10) comportant à une de ses extrémités un dispositif de guidage (20) d'une aiguille selon l'une des revendications 1 à 7.

9. Bras robotisé (10) selon la revendication 8 comportant une unité de contrôle connectée au dispositif de guidage (20) d'une aiguille et déterminant, sur la base d'informations transmises par le système de navigation optique dudit dispositif de guidage (20) d'une aiguille, la position des premier mors (30) et second mors (40) l'un par rapport à l'autre.

10. Bras robotisé (10) selon la revendication 9 dans lequel, lorsque le dispositif de guidage (20) d'une aiguille est selon les revendications 6 et 7, l'unité de contrôle est apte à déterminer, sur la base des informations reçues par le module de lecture, la position de la paire d'éléments optiques de référence par rapport à l'élément optique de référence individuel et d'en déduire la position des premier mors (30) et second mors (40) l'un par rapport à l'autre.

11. Bras robotisé (10) selon la revendication 9 dans lequel l'unité de contrôle est configurée pour piloter les déplacements du bras robotisé (10), et donc les déplacements du guide aiguille (22), en fonction de la position des premier mors (30) et second mors (40) l'un par rapport à l'autre.

12. Bras robotisé (10) selon la revendication 11 dans lequel l'unité de contrôle est configurée pour interdire tout déplacement du bras robotisé (10) lorsqu'elle détermine que les premier mors (30) et second mors (40) sont en position de guidage.

13. Bras robotisé (10) selon la revendication 11 dans lequel l'unité de contrôle est configurée pour autoriser le déplacement du bras robotisé (10) lorsqu'elle détermine que les premier mors (30) et second mors (40) sont en position de dégagement.

## Patentansprüche

1. Führungsvorrichtung (20) einer Nadel, die einen Werkzeughalter (21) aufweist, der zum Befestigen an dem Ende eines Roboterarms (10) zur medizinischen Unterstützung bestimmt ist, wobei der Werkzeughalter (21) eine Nadelführung (22) trägt, wobei die Nadelführung (22) eine erste Backe (30) und eine zweite Backe (40) aufweist, die jeweils mit einer Nut (35, 45) versehen sind, wobei sich die Nuten entlang paralleler Längsachsen erstrecken, wobei die erste Backe (30) und die zweite Backe (40) von dem Werkzeughalter so getragen werden, dass eine Drehbewegung der ersten Backe (30) und zweiten Backe (40) zueinander zwischen einer "Führungsposition", in der die Nuten (35, 45) aneinander angrenzen und einen Führungskanal (23) einer Nadel definieren, und einer "Freigabeposition", in der die Nuten (35, 45) voneinander entfernt sind und einen seitlichen Freigabebereich einer Nadel definieren, zugelassen wird,
**dadurch gekennzeichnet, dass** die Führungsvorrichtung (20) ein optisches Navigationssystem aufweist, das dazu bestimmt ist, mit einer Steuereinheit des Roboterarms (10) verbunden zu werden, die basierend auf Informationen, die von dem optischen Navigationssystem übertragen werden, die Position der ersten Backe (30) und der zweiten Backe (40) zueinander bestimmt.

2. Führungsvorrichtung (20) einer Nadel nach Anspruch 1, wobei das optische Navigationssystem optische Referenzelemente aufweist.

3. Führungsvorrichtung (20) einer Nadel nach Anspruch 2, wobei die optischen Referenzelemente Kugeln sind, deren Oberfläche reflektierend ist.

4. Führungsvorrichtung (20) einer Nadel nach Anspruch 3, wobei die Kugeln von Schenkeln getragen werden, die sich von einem oberen Ende (31, 41) jeder der ersten Backe (30) und zweiten Backe (40) erstrecken.

5. Führungsvorrichtung (20) einer Nadel nach einem der Ansprüche 2 bis 4, wobei die erste Backe (30) ein einzelnes optisches Referenzelement und die zweite Backe (40) mindestens ein Paar optische Referenzelemente aufweist.

6. Führungsvorrichtung (20) einer Nadel nach Anspruch 5, wobei das Paar optischer Referenzelemente von einem Paar Schenkeln getragen wird, das sich von einem gemeinsamen Schenkel erstreckt, durch den sie an der zweiten Backe (40) befestigt sind.

7. Führungsvorrichtung (20) einer Nadel nach einem der Ansprüche 2 bis 6, wobei das optische Navigationssystem ein Lesemodul aufweist, das dazu bestimmt ist, der Steuereinheit Informationen über die räumliche Position jedes optischen Referenzelements zu übermitteln.

8. Roboterarm (10), der an einem seiner Enden eine Führungsvorrichtung (20) einer Nadel nach einem der Ansprüche 1 bis 7 aufweist.

9. Roboterarm (10) nach Anspruch 8, der eine Steuereinheit aufweist, die mit der Führungsvorrichtung (20) einer Nadel verbunden ist und basierend auf Informationen, die von dem optischen Navigationssystem der Führungsvorrichtung (20) einer Nadel übermittelt werden, die Position der ersten Backe (30) und der zweiten Backe (40) zueinander bestimmt.

10. Roboterarm (10) nach Anspruch 9, wobei, wenn die Führungsvorrichtung (20) einer Nadel nach Anspruch 6 und 7 ist, die Steuereinheit in der Lage ist, basierend auf den von dem Lesemodul empfangenen Informationen die Position des Paars optischer Referenzelemente in Bezug auf das einzelne optische Referenzelement zu bestimmen und daraus die Position der ersten Backe (30) und der zweiten Backe (40) zueinander abzuleiten.

11. Roboterarm (10) nach Anspruch 9, wobei die Steuereinheit so eingerichtet ist, dass sie die Bewegungen des Roboterarms (10) und damit die Bewegungen der Nadelführung (22) in Abhängigkeit von der Position der ersten Backe (30) und der zweiten Backe (40) zueinander steuert.

12. Roboterarm (10) nach Anspruch 11, wobei die Steuereinheit so eingerichtet ist, dass sie jede Bewegung des Roboterarms (10) verhindert, wenn sie bestimmt, dass sich die erste Backe (30) und die zweite Backe (40) in der Führungsposition befinden.

13. Roboterarm (10) nach Anspruch 11, wobei die Steuereinheit so eingerichtet ist, dass sie die Bewegung des Roboterarms (10) zulässt, wenn sie bestimmt, dass sich die erste Backe (30) und die zweite Backe (40) in der Freigabeposition befinden.

## Claims

1. Device (20) for guiding a needle comprising a tool holder (21) intended to be attached to the end of a robotic arm (10) for medical assistance, said tool holder (21) supporting a needle guide (22), said needle guide (22) comprising a first jaw (30) and a second jaw (40) respectively comprising a groove (35, 45), said grooves extending along parallel longitudinal axes, said first jaw (30) and second jaw (40) being supported by the tool holder so as to allow the rotational movement of said first jaw (30) and second jaw (40) relative to one another between a so-called "guide position", in which the grooves (35, 45) are adjacent and define a guide channel (23) of a needle, and a so-called "disengaged position" in which the grooves (35, 45) are spaced apart from one another and define a needle lateral disengagement zone,
**characterized in that** the guide device (20) comprises an optical navigation system intended to be connected to a control unit of the robotic arm (10) determining, based on information transmitted by said optical navigation system, the position of the first jaw (30) and second jaw (40) relative to one another.

2. Device (20) for guiding a needle according to claim 1, wherein the optical navigation system comprises optical reference elements.

3. Device (20) for guiding a needle according to claim 2, wherein the optical reference elements are spheres with a reflective surface.

4. Device (20) for guiding a needle according to claim 3, wherein the spheres are supported by arms extending from an upper end (31, 41) of each of the first jaw (30) and second jaw (40).

5. Device (20) for guiding a needle according to any one of claims 2 to 4, wherein the first jaw (30) comprises an individual optical reference element and the second jaw (40) comprises at least one pair of optical reference elements.

6. Device (20) for guiding a needle according to claim 5, wherein the pair of optical reference elements is supported by a pair of arms extending from a common arm by which they are attached to the second jaw (40).

7. Device (20) for guiding a needle according to any one of claims 2 to 6, wherein the optical navigation system comprises a reading module for transmitting to the control unit information relating to the spatial position of each optical reference element.

8. Robotic arm (10) comprising, at one of its ends, a device (20) for guiding a needle according to any one of claims 1 to 7.

9. Robotic arm (10) according to claim 8, comprising a control unit connected to the device (20) for guiding a needle and determining, based on information transmitted by the optical navigation system of said device (20) for guiding a needle, the position of the first jaw (30) and second jaw (40) relative to one another.

10. Robotic arm (10) according to claim 9, wherein, when the device (20) for guiding a needle is according to claims 6 and 7, the control unit is able to determine, based on the information received by the reading module, the position of the pair of optical reference elements relative to the individual optical reference element and to deduce therefrom the position of the first jaw (30) and second jaw (40) relative to one another.

11. Robotic arm (10) according to claim 9, wherein the control unit is configured to control the movements of the robotic arm (10), and therefore the movements of the needle guide (22), according to the position of the first jaw (30) and second jaw (40) relative to one another.

12. Robotic arm (10) according to claim 11, wherein the control unit is configured to prohibit any movement of the robotic arm (10) when it determines that the first jaw (30) and second jaw (40) are in the guiding position.

13. Robotic arm (10) according to claim 11, wherein the control unit is configured to allow the movement of the robotic arm (10) when it determines that the first jaw (30) and second jaw (40) are in the disengaged position.
